# EUROPÄISCHE PATENTANMELDUNG

(11) **EP 2 158 886 A2**
(43) Veröffentlichungstag der Anmeldung: **03.03.2010**
(21) Anmeldenummer: 09010909.1
(22) Anmeldetag: 26.08.2009
(51) Int. Cl.: A61F 13/08, A61F 13/00

(54) **Strumpf oder Bandage mit höherer Haltbarkeit**

(30) Priorität: 27.08.2008 DE 102008039853
(71) Anmelder: OFA Bamberg GmbH, 96052 Bamberg (DE)
(72) Erfinder: Gebuhr, Helmut, 96047 Bamberg (DE)
(74) Vertreter: Lenzing Gerber Stute

(57) **Zusammenfassung**

Die Erfindung betrifft einen Strumpf oder Bandage, ein elastisches Umwindegarn (1, 10 100) aufweisend, wobei das Umwindegarn (1, 10 100) mindestens einen Kernfaden (2, 12, 102) und mindestens einen Umwindefaden aufweist, wobei der elastische Kernfaden (2, 12, 102) einen Bestandteil aus Multiester oder auf Olefin-Basis aufweist oder vollständig durch ein Multiester oder einem Material auf Olefin-Basis gebildet ist.

## Beschreibung

Die vorliegende Erfindung betrifft einen Strumpf oder Bandage, ein elastisches Umwindegarn aufweisend.

Bisherige gummielastische Strümpfe sind aus umsponnenen Garnen hergestellt, deren Kern aus einem elastischen Material, in der Regel Elastan oder Elastodien, gefertigt ist. Diese bislang verwendeten Materialien haben den Nachteil, dass sie nur bedingt hitzebeständig sind. Werden diese Materialien für insbesondere Anti-Thrombose Strümpfe in Krankenhäusern und Kliniken verwendet, so geht aufgrund der verwendeten Temperaturen zur Desinfektion der Strümpfe deren Elastizität in der Regel nach zehn Reinigungsvorgängen verloren. Manche Kliniken verwenden die Strümpfe bis zu zwanzig Wäschen und erreichen die verlängerte Haltbarkeit durch eine verringerte Temperatur oder Trockenzeit beim Trocknungsvorgang. Die EN 556 für die Sterilisation von Medizinprodukten schreibt für den Trocknungsvorgang vor, dass dieser bei 121°C ca. 15 - 20 min oder bei 134°C ca. 5 - 10 min andauern muss. In der Regel werden Trommeltrockner, ähnlich Haushaltstrocknern verwendet.

Beim Reinigungsvorgang hat der Trocknungsvorgang, im Gegensatz zum Waschvorgang mit Zusatz von Essigsäuren, den größten Einfluss auf die Lebensdauer der verwendeten Materialien. In der Praxis werden beim Trocknungsvorgang Temperaturen von bis zu 134°C erreicht, können aber auch bis zu 160°C betragen.

Wichtig für die Sterilisation ist, dass die Chemikalien beim Spülvorgang am Schluss vollständig heruntergelöst werden und dass die Strümpfe nicht übertrocknet werden.

Wie bereits beschrieben, enthalten Anti-Thrombosestrümpfe in den meisten Fällen Elastan. Elastan hat eine Formtemperatur von 130°C. Bei 170°C wird die Verklebungstemperatur erreicht. Aufgrund dessen wird das Elastan nach wenig Wäschen und Trockenvorgängen spröde und verliert seine elastischen Eigenschaften, womit das Rückzugverhalten des Elastans deutlich verschlechtert wird und der Strumpf ausleiert. Die medizinische Wirksamkeit bzgl. des ursprünglichen Druckverhaltens (Druck und Druckverlauf) wird spätestens nach 20 Wäschen nicht mehr eingehalten.

Wird Elastodien verwendet, so ist die Lebensdauer noch geringer, da dessen Verklebungstemperatur lediglich 120°C beträgt.

Ebenso wirken Hautschweiß und Waschchemikalien lebensdauerverkürzend auf das Elastan bzw. Elastodien.

Aufgabe der vorliegenden Erfindung ist es daher, einen Anti-Thrombose-Strumpf bereitzustellen, der eine größere Lebensdauer aufweist.

Diese Aufgabe wird erfindungsgemäß dadurch gelöst, dass das Umwindegarn mindestens einen Kernfaden und mindestens einen Umwindefaden aufweist, wobei der elastische Kernfaden einen Bestandteil aus Multiester oder auf Olefin-Basis aufweist oder vollständig durch ein Multiester oder einem Material auf Olefin-Basis gebildet ist. Insbesondere ist es von Vorteil, wenn die bekannten Garne LYCRA T400® oder DOW XLA® als elastisches Umwindegarn, insbesondere als elastischer Einlegefaden verwendet wird.

Damit die Haltbarkeit, insbesondere die Temperaturbeständigkeit nicht durch das Material des mindestens einen Umwindefadens gemindert wird, kann für den mindestens einen Umwindefaden ebenfalls das Material des Kernfadens verwendet werden. Der mindestens eine Umwindefaden sollte daher überwiegend oder vollständig aus einem Multiester oder auf Olefin-Basis gefertigt sein. Gleichsam ist es jedoch auch möglich, dass der mindestens eine Umwindefaden vollständig oder teilweise aus Polyamid, insbesondere PA 6 oder PA 6.6, oder Polyester besteht, da auch diese Materialien eine gute Hitzebeständigkeit aufweisen.

Bei dem Garn "Dow XLA"® handelt es sich um ein Material auf Olefinbasis und weist Lastol auf, womit eine gummielastische Faser herstellbar ist. Gemäß Römpp Chemielexikon handelt es sich bei Olefinen um einfach ungesättigte lineare od. verzweigte aliphat. Kohlenwasserstoffe CnH2n.

Das Garn LYCRA T400® ist aus einem Elastomultiester und weist eine Bikomponentenfaser aus Polyester auf.

Vor kurzem wurde die Textilie "Elastomultiester" neu als Nr. 45 in die Anlage 1 des Textilkennzeichnungsgesetzes aufgenommen. Unter einem Elastomultiester versteht man eine Faser aus zwei oder mehr chemisch verschiedenen linearen Makromolekülen.

Ein "Elastomultiester" ist definiert durch:
"Fasern, die durch die Interaktion von zwei oder mehreren chemisch verschiedenen linearen Makromolekülen in zwei oder mehreren verschiedenen Phasen entstehen (von denen keine 85 Gewichtsprozent übersteigt), die als wichtigste funktionale Einheit Estergruppen enthalten (zu mindestens 85%) und die nach geeigneter Behandlung nach einer Dehnung um die anderthalbfache ursprüngliche Länge sofort wieder nahezu in ihre Ausgangslage zurückkehren, wenn sie entlastet werden."

Sowohl "LYCRA T400®" als auch "DOW XLA®" weisen eine deutlich verbesserter Langlebigkeit auf, da beide Fäden die Eigenschaften von Polyester aufweisen und folgende Eigenschaften aufweisen:
- Hitzebeständigkeit bis 220°C bei 2 - 5 min
- Chlorresistent/Säure- und Alkalibeständig
- Beständigkeit gegen UV/Xenonlicht
- Weichere Dehnungskurve als Elastan - damit ausgezeichnete Dimensionsstabilität - keine übermäßige Restschrumpfkraft - Stretch bleibt erhalten

Zwar werden bereits heute die Materialien Lastol bzw. Polyester für elastische Bündchen in Arbeitsbekleidung, Medizinalbekleidung, Socken und Sportbekleidung eingesetzt. Hierbei handelt es sich jedoch um Nacktfäden, d.h. nicht um Umwindegarne bestehend aus Kernfaden und Umwindungsfaden.

Der erfindungsgemäße Strumpf ist insbesondere ein medizinischer Strumpf mit Kompression, insbesondere ein Thrombose-Propyhlaxe-Strumpf, welcher aufgrund der hohen Trockentemperaturen bisher nur eine geringe Lebensdauer erreicht. Die Erfindung sieht vor, das bisher verwendete Elastan bzw. Elastodien durch die neue Fasergattung der Olefine bzw. des neuen Polyestertypes zu ersetzen. Hierdurch ergibt sich vorteilhaft eine wesentlich höhere Lebensdauer bzw. Haltbarkeit, in erster Linie aufgrund der höheren Temperaturbeständigkeit, bedingt durch die mehr als 50°C höhere Formtemperatur. Die Anzahl der möglichen Waschzyklen wird damit vorteilhaft erhöht.

Für einen medizinischen Thrombose-Prophylaxe-Strumpf muss beispielhaft ein Druck an der Fessel von 18 - 21 mmHG sowie der zusätzliche medizinische Druckverlauf erreicht werden. Dies entspricht einem Druck der Kl. 1 gemäß RAL - GZ 387 für medizinische Kompressionsstrümpfe. Der Strumpf erhält, wie marktüblich, einen eingelegten gummielastischen Faden. Dieser Einlegefaden kann nach der Erfindung folgendermaßen konstruiert sein:

Der Kernfaden des als Umwindungsgarn ausgebildeten Einlegefadens ist durch die von Dow Chemical vertriebene "Dow XLA®"-Faser oder die von Invista unter der Marke "LYCRA T400®" vertriebene Faser gebildet. Die derzeit maximalen Kernstärken sind 156dtex bei Dow XLA® bzw. 660dtex bei LYCRA T400®. Aufgrund des größeren Stretches wird vorteilhaft "Dow XLA®" als Kernfaden verwendet. Vorteilhaft sind alle Elastane bzw.

Elastodien Komponenten durch die Fasergattung aus Olefin bzw. Polyolefin oder Multiester ersetzt. Die sonstigen Materialkomponenten sollten, wie üblich aus Polyamid, vorzugsweise PA6 (Schmelzpunkt ca. 215°C) oder PA6.6 (Schmelzpunkt ca. 255°C) oder Polyester (Erweichungstemperatur ca. 235°C, Schmelzpunkt 250°C) bestehen. Es können aber auch andere Materialien gewählt werden. Wichtig ist, dass deren Beständigkeit bzgl. Haltbarkeit mindestens der der Polyolefin-Faser entspricht.

Ebenso muss die Herstellung für das erfindungsgemäße Umwindegarn sowie das Strickprogramm zum Verstricken des Umwindegarns angepasst werden. Da das Olefin im Gegensatz zu Elastan oder Elastodien eine andere Anordnung der amorphen und kristallinen Bereiche und dadurch eine flachere Kraft-Dehnungskurve besitzt, insbesondere im unbehandelten Zustand, muss insbesondere beim Einlegefaden, der die Kompression erzeugt, folgendes beachtet werden:

Bei der Herstellung des Umwindefadens ist ein höherer Verzug notwendig, da die Rücksprungkraft des Dow XLA - im nicht aktivierten Zustand - grundsätzlich geringer ist, im Vergleich zu z.B. Lycra oder Gummi. Dies ist auch in Abhängigkeit von der Gesamtfadenkonstruktion zu sehen. Der Verzug gibt an, um wie viel der Kern beim Umwindeprozess verstreckt wird. Hier kann es sich um das doppelte, im Vergleich zu Elastan, handeln.

Beim Strickvorgang ist folgendes zu berücksichtigen:

Der Faden muss grundsätzlich durch die Fournisseure weniger verzogen werden. Der Strumpf erhält dadurch mehr Breite. Der Grund ist folgender: Der Schrumpf beim Färben/Waschen, z.B. bei 95°C, ist bei Elastan mit ca. 10% deutlich kleiner als bei Olefin mit ca. 50%. Erst nach dem Schrumpf entfaltet das Olefin seine elastischen Eigenschaften.

Nachfolgend wird anhand von Zeichnungen das erfindungsgemäße Umwindungsgarn sowie ein Gestrick für einen medizinischen Strumpf oder eine Bandage näher erläutert.

Es zeigen:
- Fig. 1:: Eine erste Ausführungsform eines erfindungsgemäßen Umwindegarns;
- Fig. 2:: eine zweite Ausführungsform eines erfindungsgemäßen Umwindegarns mit zwei Kernfäden;
- Fig. 3:: eine dritte Ausführungsform eines erfindungsgemäßen Umwindegarns mit einem Kernfaden umwickelt mit zwei Umwindefäden;
- Fig. 4:: eine erste mögliche Ausführungsform eines Gewirks für einen Strumpf oder eine Bandage;
- Fig. 5:: eine zweite mögliche Ausführungsform eines Gewirks für einen Strumpf oder eine Bandage.

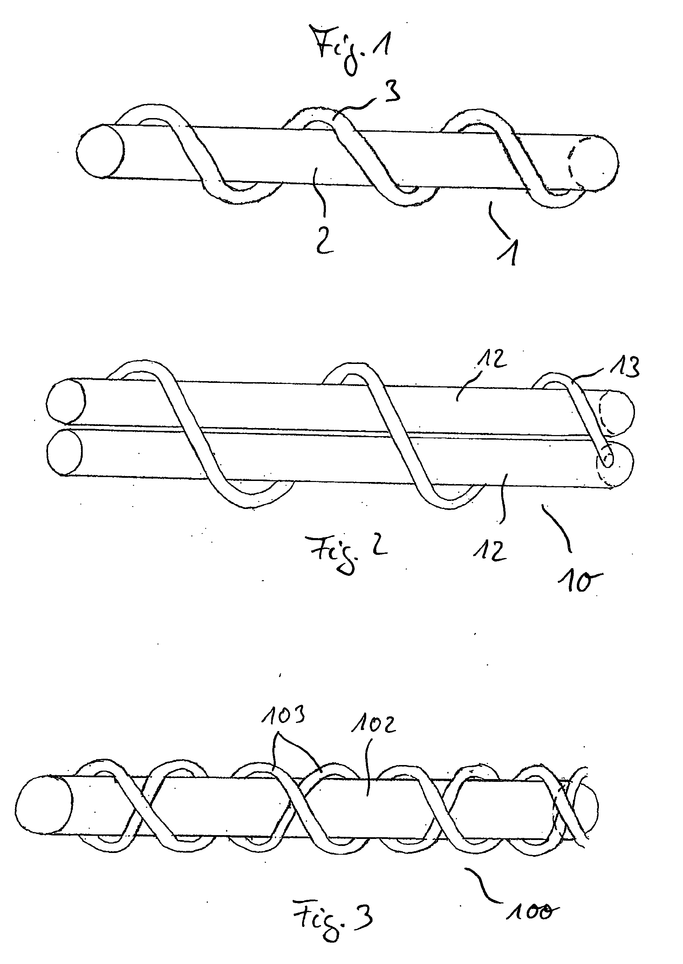

Die Figur 1 zeigt eine erste mögliche Ausführungsform eines Umwindegarns für einen erfindungsgemäßen Strumpf oder Bandage. Das Umwindegarn 1 weist einen Kernfaden 2 auf, der von einem Umwindefaden 3 umwickelt ist. Der Kernfaden 2 ist aus einem Material, welches identisch oder ähnlich ist wie die "Dow XLA®"-Faser oder die "LYCRA T400®"-Faser. Der Umwindefaden 3 kann ebenfalls aus einem der beiden vorgenannten Materialien oder Polyamid, vorzugsweise PA 6 (Schmelzpunkt ca. 215°C), PA6.6 (Schmelzpunkt ca. 255°C) oder Polyester gebildet sein. Ebenso ist es möglich, das der Kernfaden 2 aus einer Mischung mit einem Anteil von aus einem auf Olefin-Basis oder Multiester hergestellten Material besteht. Gleiches gilt für den Umwindefaden.

Die Figur 2 zeigt eine zweite mögliche Ausgestaltung eines Umwindegarns 10 zur Herstellung eines Strumpfes oder einer Bandage, bei dem zwei Kernfäden 12 und ein Umwindefaden 13 vorgesehen sind. Die einzelnen Fäden sind aus den gleichen Materialien wie sie bereits für das Umwindegarn gem. Figur 1 beschrieben sind. Es ist ebenso im Sinne der Erfindung mehr als zwei Kernfäden und mehr als einen Umwindefaden vorzusehen.

Die Figur 3 zeigt ein Umwindegarn 100, bestehend aus einem Kernfaden 102, welcher mit zwei Umwindefäden 103 umwickelt ist.

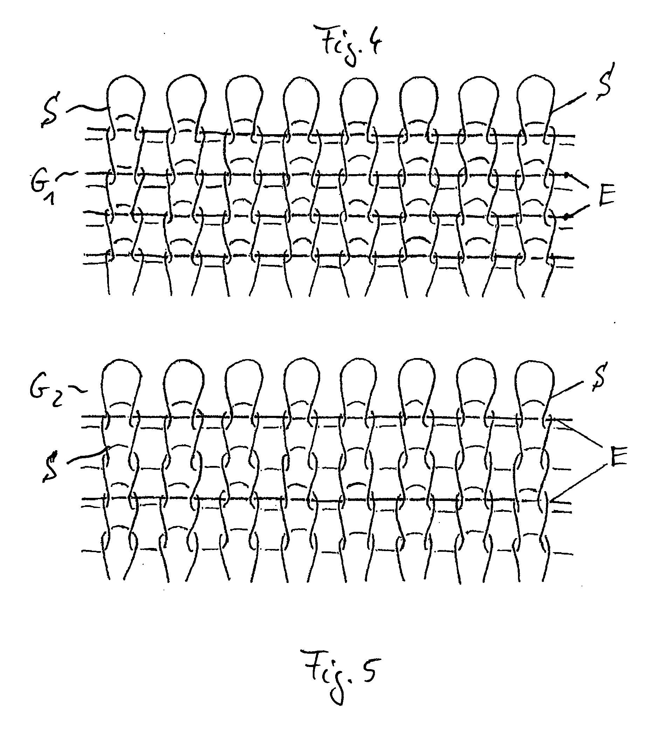

Die Figur 4 zeigt ein erstes mögliches Gewirk G₁ zur Herstellung eines erfindungsgemäßen Strumpfes oder einer Bandage. Es handelt sich dabei um eine Zwei-Zugkonstruktion, welche sowohl gummielastisch in Quer- als auch in Längsrichtung ist, und die insbesondere für den Fuß- und/oder das Beinteil verwendbar ist. Der Einlegefaden ist ein Faden, wie er z.B. in den Figuren 1 bis 3 beschrieben und dargestellt ist. Vorzugsweise besteht der Einlegefaden aus einem Umwindegarn mit zwei oder drei parallel gelegten Kernfäden, z.B. aus Dow XLA 156 dtex, welche mit einer Einfachumwindung umwickelt sind. Abhängig von der benötigten und zu erzielenden Kompression können auch mehr als drei Kernfäden parallel gelegt werden. Genauso ist eine Doppelumwindung denkbar oder alternative Umspinnungsverfahren. Beim Herstellungsprozess kann bekanntermaßen durch verschiedene Verzüge, Tourenzahlen und/oder Umwindematerial ebenso die Kraft- und Dehnung beeinflusst werden.

Der Strickfaden S weist vorteilhaft ebenfalls einen gummielastischen Strickfadenkern aus einem Material, welches identisch oder ähnlich ist wie die "Dow XLA®"-Faser oder die "LYCRA T400®"-Faser. Es kann durch die Fadenstärken, vorzugsweise 44
- 84 dtex ersetzt werden. Der Kern ist umsponnen, wobei die Umwindungen aus einem anderen Material oder Materialzusammensetzungen bestehen können. Die Umspinnung ist vollständig oder teilweise aus Polyamid, insbesondere PA 6 oder PA 6.6, oder Polyester, da auch diese Materialien eine gute Hitzebeständigkeit aufweisen.

Auch das Fußbündchen (off. Spitze, Inspektionsöffnung) bzw. der Abschlussrand (Bein), Fußspitze (geschlossene Spitze) und Ferse können aus dem vorbeschriebenen Gewirk gefertigt werden.

Das Gestrick G₁, G₂ ist ein Rechts/Links-Gestrick mit eingelegtem elastischem Faden, welcher in jeder oder in jeder zweiten Reihe, wie in Figur 5 dargestellt, eingearbeitet ist. Genauso kann auch in jeder x-ten Reihe ein Schussfaden eingelegt werden, um mehr Dehnung zu erhalten.

## Patentansprüche

1. Strumpf oder Bandage, ein elastisches Umwindegarn (1, 10 100) aufweisend, **dadurch gekennzeichnet, dass** das Umwindegarn (1, 10 100) mindestens einen Kernfaden (2, 12, 102) und mindestens einen Umwindefaden aufweist, wobei der elastische Kernfaden (2, 12, 102) einen Bestandteil aus Multiester oder auf Olefin-Basis aufweist oder vollständig durch ein Multiester oder einem Material auf Olefin-Basis gebildet ist.

2. Strumpf oder Bandage nach Anspruch 1, **dadurch gekennzeichnet, dass** der mindestens eine Kernfaden (2, 12, 102) aus dem Material LYCRA T400® oder DOW XLA® ist.

3. Strumpf oder Bandage nach Anspruch 1 oder 2, **dadurch gekennzeichnet, dass** der mindestens eine Umwindefaden (3, 13, 103) überwiegend oder vollständig aus einem Multiester oder auf Olefin-Basis gefertigt ist.

4. Strumpf oder Bandage nach Anspruch 1 oder 2, **dadurch gekennzeichnet, dass** der mindestens eine Umwindefaden (3, 13, 103) vollständig oder teilweise aus Polyamid, insbesondere PA 6 oder PA 6.6, oder Polyester besteht.

5. Strumpf oder Bandage nach einem der vorhergehenden Ansprüche, **dadurch gekennzeichnet, dass** das Umwindegarn (1, 10, 100) als Einlegfaden bzw. Schussfaden in einem Gewirk (G₁, G₂) eingearbeitet ist.

6. Strumpf oder Bandage nach Anspruch 5, **dadurch gekennzeichnet, dass** der Strumpf oder die Bandage zumindest bereichsweise, insbesondere der Fuß- und/oder das Beinteil, aus einem Gestrick (G₁, G₂) gefertigt ist, welches insbesondere ein Rechts/Links-Gestrick mit eingelegtem Umwindegarn (1, 10 100) ist.

7. Strumpf oder Bandage nach einem der vorhergehenden Ansprüche, **dadurch gekennzeichnet, dass** der eingelegte Faden aus dem Umwindegarn (1, 10, 100) in jeder, jeder zweiten oder in jeder dritten Reihe eingelegt ist.
